# EUROPEAN PATENT SPECIFICATION

(11) **EP 1 778 325 B1**
(45) Date of publication and mention of the grant of the patent: **28.07.2010**
(21) Application number: 05758800.6
(22) Date of filing: 27.06.2005
(51) Int. Cl.: A61M 16/00

(54) **DELTA FLOW PRESSURE REGULATOR**
DELTAFLUSS-DRUCKREGLER
REGULATEUR DE LA PRESSION D'UN FLUX DELTA

(30) Priority: 08.07.2004 SE 0401809; 15.09.2004 US 610137 P
(43) Date of publication of application: 02.05.2007
(73) Proprietor: Breas Medical AB, 435 33 Mölnlycke (SE)
(72) Inventor: TIEDJE, Mikael, S-425 30 Hisings Backa (SE)
(74) Representative: Valea AB
(86) International application number: PCT/EP2005/006880
(87) International publication number: WO 2006/005432

(56) References cited:
- WO-A-02/28281
- FR-A- 2 695 320
- US-A- 5 660 171
- US-A1- 2004 050 387
- US-B1- 6 305 374

## Description

### Field of the invention

The present invention relates to the measurement and control of breathing gas administration into humans, and more specifically the invention relates to a system for rapid response to changes of pressure/flow of breathing gas using delta flow control.

### Background of the invention

Patients suffering from different forms of breathing disorders can be subject to several types of treatments depending on the illness or disorder present. Such treatments include surgical procedures, pharmacologic therapy, and non-invasive mechanical techniques. Surgical techniques to remedy breathing disorders constitute a considerable risk for the patient and can lead to permanent injury or even mortality. Pharmacologic therapy has in general proved disappointing with respect to treating certain breathing disorders, e.g. sleep apnea. It is therefore of interest to find other treatments, preferably non-invasive techniques.

A mechanical ventilator represents a non-invasive technique for treatment of certain breathing disorders such as ventilatory failure, hypoventilation, and periodic breathing during sleep and awake and in sleep apnea that occurs exclusively during sleep. Ventilatory failure includes all forms of insufficient ventilation with respect to metabolic need whether occurring during wake or periods of sleep. Hypoventilation and periodic breathing, in its most frequently occurring form referred to as Cheyne-Stokes ventilation, may occur periodically or constantly during wake or sleep. Conditions associated with hypoventilation, in particular nocturnal hypoventilation include e.g. central nervous system disorders such as stroke, muscular dystrophies, certain congenital conditions, advanced chronic obstructive pulmonary disease (COPD), etc. Cheyne-Stokes ventilation or various forms of central apnea are commonly associated with cardiac and circulatory disorders, in particular cardiac failure.

Sleep apnea can be categorized into two different forms that occur selectively or in combination. In central sleep apnea, a primarily central nervous system coordination disorder, all respiratory movement is interrupted leading to a sleep apnea event. Obstructive sleep apnea, in contrast, is associated with upper airway collapse, presumably caused by loss of or inadequate upper airway muscle tone. This condition is particularly likely to occur in subjects with narrow upper airways due to excess soft tissue or anatomical abnormalities. Obstructive sleep apnea is the most common type of sleep apnea event. Apnea events are considered pathological, for instance, if they exceed 10 seconds in duration and if they occur more frequently than 10 times per hour of sleep.

Ventilatory failure is a potentially life threatening condition. The general comorbidity in patients with failing ventilation is considerable. The condition is highly disabling in terms of reduced physical capacity, cognitive dysfunction in severe cases and poor quality of life. Patients with ventilatory failure therefore experience significant daytime symptoms but in addition, the majority of these cases experience a general worsening of their condition during state changes such as sleep. The phenomenon of disordered breathing during sleep, whether occurring as a consequence of ventilatory failure or as a component of sleep apnea in accordance with the description above causes sleep fragmentation. Daytime complications include sleepiness and cognitive dysfunction. Severe sleep disordered breathing occurring in other comorbid conditions like obesity, neuromuscular disease, post polio myelitis states, scoliosis or heart failure may be associated with considerable worsening of hypoventilation and compromised blood gas balance. Sleep apnea has been associated with cardiovascular complications including coronary heart disease, myocardial infarction, stroke, arterial hypertension, thrombosis, and cardiac arrhythmia. It is therefore of both immediate and long-term interest to reduce the exposure to sleep disordered breathing.

Recent advancement in mechanical non-invasive ventilator techniques includes administration of continuous positive airway pressure (CPAP) in different forms of sleep disordered breathing. During CPAP administration an elevated airway pressure is maintained throughout the breathing phase during a period coinciding with sleep. In sleep apnea this procedure may provide appropriate stabilization of the upper airway thereby preventing collapse. This, so called mono-level CPAP therapy, provides an almost identical pressure during inhalation and exhalation. Not only may CPAP be uncomfortable for the patient due to a sensed increased work of breathing during ventilation, specifically expiration. Some forms of apnea, mainly including those of central origin, and most forms of hypoventilation are only poorly controlled by CPAP. A more recently developed bi-level CPAP system administers different pressure levels during inhalation and exhalation. Bi-level CPAP provides increased comfort for most patients and not infrequently, an improved clinical response. Bi-level CPAP provides two pressure levels, Inspiratory Positive Airway Pressure (IPAP) and Expiratory Positive Airway Pressure (EPAP). IPAP is administered during the inhalation phase while EPAP is given during the exhalation phase.

In a Bi-Level or CPAP breathing apparatus used for treatment of sleeping disorders as e.g. hypoventilation especially nocturnal hypoventilation, e.g. central nervous system disorders such as muscular dystrophies, Chronic obstructive pulmonary disorder (COPD) etc, the pressure sensing device for regulating the breathing pressure is normally located inside the breathing apparatus to avoid long measuring tubes and risks with e.g. kinked, wrongly connected sensing device or connector leakage.

Since the patient and user requirement for higher accuracy is an increasing demand, it is essential to implement a system for higher accuracy in Bi-Level and CPAP breathing devices.

To solve this pressure regulating problem a system that uses predetermined tube compensation for pressure losses in breathing tube (patient circuit) can be used. However such a system will lack in flexibility due to this predetermination which leads the user / patient to use one or a couple of patient circuits. By using a measuring tube in parallel to the patient breathing circuit, the pressure accuracy may be increased but the risk for kinked tubing is increased together with an increased necessity for cleaning the tubing as well. One other way to increase pressure measuring accuracy may be utilized by feeding the measuring tube inside the patient breathing circuit. This latter method will however reduce the active breathing tube diameter and hence reduce the breathing flow of gas. The necessity for cleaning the measuring tube and breathing circuit will also be increased since there are more surfaces inside the breathing gas flow.
<Page3a>

### Summary of the invention

It is an object of the present invention to provide a system that remedies the above mentioned problems. During pressure regulation when using only a pressure sensor as input, the patient's airways and the breathing tube has to be fully saturated with breathing gas before the pressure sensor can recognize Several solutions have been documented for finding a triggering point between different breathing phases for which flow triggering solutions are used. For instance this has been shown in US6305374, FR2695320, US20040050387. and US5660171. However, these documents are not specifically concerned problems related to holding pressure levels constant.
changes in pressure. The time lag between patient effort and the breathing apparatus response in such a system will create a pressure drop in inhalation and a pressure peak in exhalation which creates pressure not being regulated within requirements, creating a discomfort for the patient.

Since gas flow in breathing tubes and breathing masks at low pressures (< 30 cmH₂O ) may be considered as homogenous, there is little time difference between patient breathing and actual flow sensor response. By applying a derivative signal analysis on the measured breathing gas flow, the delta energy term can be retrieved from the gas flow. This delta energy is proportional to pressure withdrawn or added into the breathing circuit and can therefore be used as an input into the pressure regulating function. By incorporating this delta energy flow value into the analyzing function for regulating the pressure, the use of a separate measuring tube is not needed. This system will also reduce the dependency of breathing tube length and width since the flow sensor may be located at the ventilator side of a breathing tube.

Other advantages of the present invention may be that it is possible to keep a constant pressure level and it constitutes a much more hygienic solution.

The present invention concerns a mechanical ventilator apparatus according to claim 1.

The processing unit may be further arranged to filter the flow signals in a noise reducing filter, such as a low-pass filter, prior to analyzing the flow signals.

The sensing means for sensing flow signals may be arranged at a mechanical ventilator side of tubing for supplying breathing gas.

The measured data may be preprocessed and preformatted prior to filtering the measured signal data.

The processing unit may further be arranged to apply the flow curve to a filtering procedure in order to reduce noise.

Another embodiment of the present invention concerns a computer program according to claim 5.

The computer program may further be arranged to filter the flow signals prior to analyzing the flow data for flow changes in order to reduce noise.

The computer program is arranged to operate on signals obtained from sensing means, for sensing flow and pressure signals, arranged at a mechanical ventilator side of tubing for supplying breathing gas.

The measured data may be preprocessed and preformatted prior to filtering the measured signal data.

### Brief description of the drawings

In the following the invention will be described in a non-limiting way and in more detail with reference to exemplary embodiments illustrated in the enclosed drawings, in which:
Fig. 1 is a schematic depiction of a ventilatory system according to the present invention.
Fig. 2 is a schematic block diagram of a ventilator apparatus according to the present invention.
Fig. 3 is a schematic diagram of air flow tubing.
Fig. 4A and 4B illustrates pressure, flow changes, and response times for inhalation and exhalation phases respectively.
Fig. 5 is a schematic illustration of a method according to the present invention.

### Detailed description of the invention

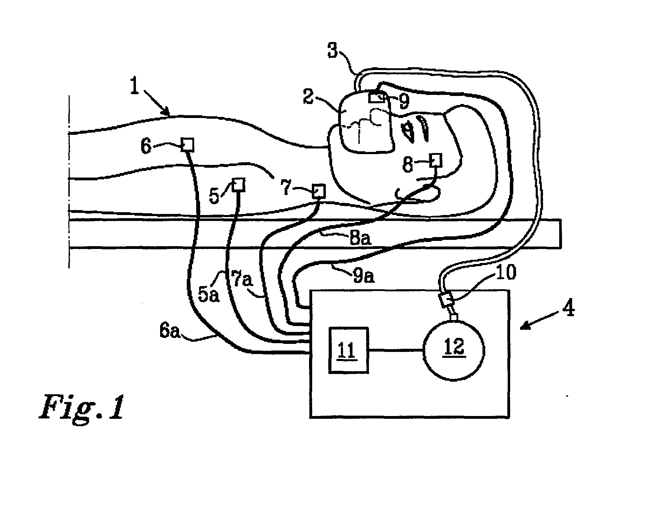

In Fig. 1 a schematic mechanical ventilation system used for the treatment of hypoventilation disorders is depicted. A ventilation system comprise a mechanical ventilator 4 supplying pressurized breathing gas, tubing 3 for guiding breathing gas to the patient 1, a breathing mask 2 or similar for administrating (supplying) the breathing gas to the patient 1, sensing means 5, 6, 7, 8, 9, and 10 for determining the physiological status of the patient 1. A mechanical ventilator 4 is supplying breathing gas for instance as a positive airway pressure via a tubing 3 and through a mask 2 to a patient 1. The mask 2 can be a face mask 2 covering both the mouth and nose or a nasal mask covering only the nose or nostrils depending on the patients needs. It can also be a hood covering the complete head or body of the patient.

The breathing gas may be of any suitable gas composition for breathing purposes as understood by the person skilled in the art, the composition depending on the physiological status of the patient.

The pressure or flow from the ventilator 4 is controlled by a processing unit 11 as shown in Fig. 1. The processing unit 11 measures one or several input parameters 5, 6, 7, 8, 9, and 10 obtained from the patient 1 describing the physiological status of the patient. Data indicative of patient status is obtained using sensors 5, 6, 7, 8, 9, and 10 connected to the patient and transferred to the processing unit 11 via connection means 5a, 6a, 7a, 8a, and 9a. These input parameters may be for instance flow or pressure signals, data obtained from EEG, EMG, EOG, and ECG measurements, O₂ and/or CO₂ measurements in relation to the patient, body temperature, blood pressure, Sp02 (oxygen saturation), eye movements, and sound measurements. It should be understood that the invention is not limited to the above mentioned input parameters but other input parameters may be used. In Fig. 1 not all sensors 5, 6, 7, 8, 9, and 10 and sensor connection means 5a, 6a, 7a, 8a, and 9a (sensor connection for sensor 10 is not shown) are depicted, only a subset is shown in order to illustrate a schematical view of the system and the depicted locations are only given as examples and are in no way limiting to the invention.

The flow sensor 10 may be located at several different positions, e.g. in the breathing air tubing 3 at any suitable position, such as close to the mechanical ventilator apparatus (or even within the ventilator housing) or in the vicinity of the mask.

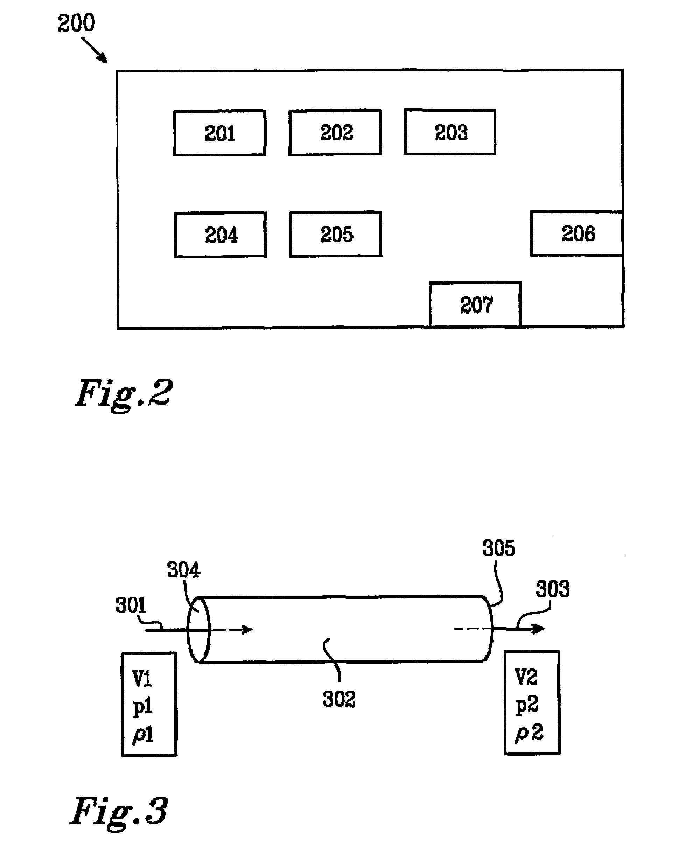

A computational device is depicted in Fig. 2. This computational device 200 is used for analyzing measured data and generating control signals to the ventilator apparatus. This computational 200 may be located within the ventilator itself or be located as a separate unit. The computational device 200 comprises at least a computational unit or processor 201 for analyzing and generating the control signals.

The computational device 200 may also have a data storage unit 202 for post analysis and inspection and there may also be a connection 207 for an external non-volatile memory device, like for instance a memory device using a USB connection, an external hard drive, a floppy disk, a CD-ROM writer, a DVD writer, a Memorystick, a Compact Flash memory, a Secure Digital memory, an xD-Picture memory card, or a Smart Media memory card. These are only given as examples, and are not limiting for the invention, many more external memory devices may be used in the invention.

The computational device 200 may also have input means 205 for manually setting control parameters and other parameters necessary for the operation of the device.

Through a communication means 206 it is possible to communicate with the device 200 to and from an external computational device (e.g. a personal computer, workstation, embedded computer, and so on as understood by the person skilled in the art) for retrieving data and results for later analysis and/or inspection. The communication means 206 can be of a serial type like for instance according to the standards RS232, RS485, USB, Ethernet, or Firewire, or of a parallel type like for instance according to the standards Centronics, ISA, PCI, or GPIB/HPIB (general purpose interface bus). The communication means 206 may also be any wireless system of the standards in the IEEE 802.11 series, HiperLAN, Bluetooth, IR, GSM, GPRS, or UMTS, or any other appropriate fixed or wireless communication system. It may also be of any proprietary non-standardized communication formats, whether being wireless or wired.

The ventilator device 4 or the computational device 200 may also have display means (not shown) for displaying measured data and obtained response parameters for use by a physician, other medical personnel, or the patient. The display means may be of any normal type as appreciated by a person skilled in the art. The data is displayed with such a high rate that a real time feedback is provided to a person monitoring the ventilator characteristics and function for immediate feedback and control.

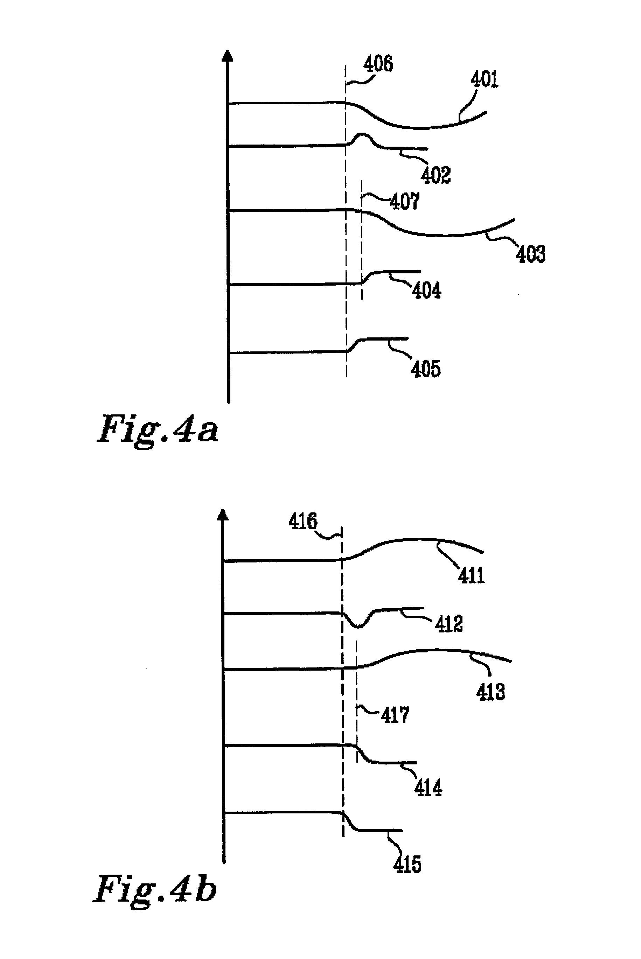

Turning now to Fig. 3 wherein a breathing gas tube 302 is schematically depicted. Since gas flow in a breathing tube 302 and mask 2 (from Fig. 1) at low pressures may be considered nearly homogenous, there is a small time lag between gas flow changes between two points in the tube 302, e.g. at the two end points 304 and 305 of the tube 302. By adding gas flow changes into a regulating function a quicker regulating response may be achieved. This means a system that faster meets the patient demand and also creates less work of breathing. A comparison of breathing responses between a standard regulating function and a system according to the present invention may be viewed in Figs 4A and 4B, with inhalation and exhalation phases respectively.

Figs 4A and 4B illustrates an actual pressure 401 and 411 in the patient 1, an actual flow change 402 and 412, a pressure sensor signal 403 and 413, a system response time using standard techniques 404 and 414, and a system response time 405 and 415 using the technique according to the present invention for the inhalation and exhalation phases respectively. Line 406 and 416 marks the start of the inhalation and exhalation phase respectively and line 407 and 417 shows when a system would have detected a pressure change triggering a regulating response in a standard system. As may be seen in Figs 4A and 4B a system using a technique according to the present invention has a much faster response time and thus the patient will experience a more comfortable breathing assistance both in the inhalation phase and the exhalation phase.

The system is utilized by placing two sensors in the air path to the patient 1 in order to monitor pressure and flow using pressure and flow sensors 9 and 10. The pressure sensor 9 senses the pressure in the patient breathing tube 3 and the pressure signal is input to the pressure regulating computational device 200 (Fig. 2). The computational device compares the pressure signal to a set pressure demand level in a pressure regulating procedure. The flow sensor 10 senses the breathing gas flow in the tube 3 and the measured signal is fed into the computational device and filtered through a low pass filter to compensate for noise, such as turbulence, electrical noise, and other noise sources. Noise may for instance be created by turbulence of the gas flow in tube or due to measurement probe inside the tube.

A flow change generating procedure is then used in order to deduce the flow changes from the flow signal and finally the flow change data is fed into the pressure regulating procedure together with the sensed pressure data. The flow change values are used as a pressure change value in the algorithm since the flow change may be considered homogenous in the tubing 3. Flow changes thus triggers the regulating algorithm to respond and change the pressure delivered to the patient 1. Many different types of regulating algorithms may be used in this application including, but not limited to, different types of PID algorithms (Proportional, Integration, and Derivative), logical function gates, and neural networks.

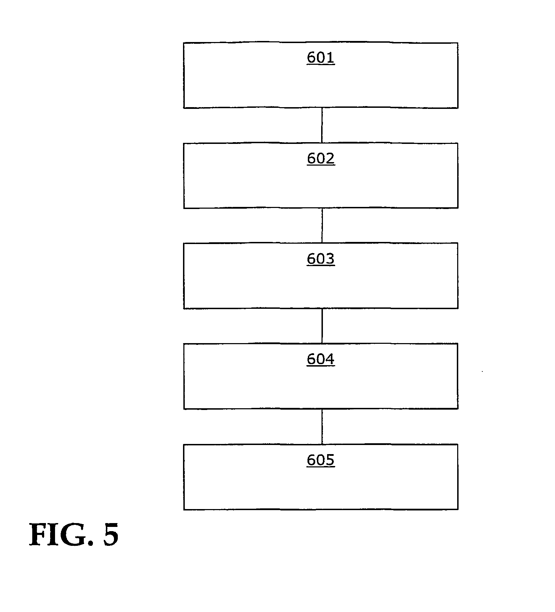

A method used by the present invention may be illustrated by Fig. 5.
1. Feeding measured pressure data into a pressure regulating procedure: 601;
2. Feeding measure flow data into a low pass filter: 602;
3. Generating flow change data from the measured and filtered flow data: 603;
4. Feeding generated flow change data into the pressure regulating procedure 604;
5. Comparing the current flow change value to a set demand pressure value: 605; and
6. Generating control signals to a mechanical ventilator apparatus in response to flow changes in order to adjust the measured pressure to the set demand pressure value: 606.

The above described method and apparatus may be used to determine many different control parameters concerning a breathing gas ventilation apparatus or method including but not limited to:
1. IPAP level
2. EPAP level
3. breathing duty cycles, including cycle rate (frequency) and time duration of different parts of the breathing duty cycle.
4. pressure limit
5. work of breathing (WOB)
6. pressure support ventilation (PSV) level
7. tidal volume (V_{T}) level
8. continuous positive airway pressure (CPAP)
9. positive end expiratory pressure (PEEP)
10. fractional inhaled oxygen concentration (Fi02) level
11. breathing gas flow rate level
12. minute ventilation level

The tidal volume (V_{T}) level is the volume of breathing gas moving in and out of the lungs per breath. PEEP is a baseline of elevated positive pressure maintained during inhalation and exhalation during machine assisted ventilation. FiO2 level is the amount of oxygen in the air inhaled. Minute ventilation level is the volume of breathing gas moving in and out of the lungs per minute.

In another preferred embodiment of the present invention the algorithms and methods are realized in a computer program residing in a computer readable medium. The computer program is arranged to acquire signals from the sensing means 5, 6, 7, 8, 9, and 10 and operating on signals indicative of gas flow and pressure in order to deduce control signals from a flow and pressure regulating algorithm, transmitting control signals to regulate the mechanical ventilator in accordance.

There are many types of ventilation modes where the above described embodiments may find its application including but not limited to:
1. Continuous positive airway pressure (CPAP),
2. Synchronized intermittent mandatory ventilation (SIMV),
3. Assist control mechanical ventilation (ACMV),
4. Pressure control ventilation (PCV),
5. Pressure support ventilation (PSV),
6. Proportional assist ventilation (PAV), and
7. Volume assured pressure support (VAPS)

These kinds of methods and devices are often used for treating disturbed breathing during for instance sleep either in the home or in a clinical environment. The methods and devices described above within the scope of the invention may also be used for treatment of many other different forms of ventilatory failure events or hypoventilation events, and treatment may be done both at home and in the clinical environment. Examples of groups of breathing disorders include, but are not limited to, breathing disorders during sleep, obstructive lung diseases (COPD), neuromuscular disorders, neurological disorders, chest wall disorders, and more.

Several benefits may be found using techniques from embodiments of the present invention: a faster response to flow changes will increase the well being of the patient 1 due to that the mechanical ventilator more accurately follows the patient 1 breathing pattern, a faster response also lowers a pressure difference between patients airway pressure (P_{aw}) and esophageous pressure (Pₑₛₒ) reducing the work of breathing (WoB). By reducing the work of breathing for the patient the risk of causing the patient anxiety is drastically lowered.

The above mentioned and described embodiments are only given as examples and should not be limiting to the present invention. Other solutions, uses, objectives, and functions within the scope of the invention as claimed in the below described patent claims should be apparent for the person skilled in the art.

## Claims

1. A mechanical ventilator apparatus (4) for supplying breathing gas to humans (1), the ventilator comprising:
- a ventilator arrangement (4);
- a processing unit (201); and
- input means for obtaining signals (5, 6, 7, 8, 9, 10) indicative of at least breathing gas flow and pressure; wherein said processing unit (201) is arranged to analyze said flow signals determining flow change values, **characterized in that** the processing unit is further arranged to determine a delta energy term from the flow changes, use said delta energy term as input in a pressure regulating procedure, and regulate said pressure against a set pressure demand value creating a constant pressure level.

2. The apparatus according to claim 1, wherein said processing unit (201) is arranged to filter said flow signals in a noise reducing filter, such as a low-pass filter, prior to analyzing said flow signals.

3. The apparatus according to claim 1, wherein said sensing means for sensing flow signals is arranged at a mechanical ventilator side of tubing (3) for supplying breathing gas.

4. The apparatus according to claim 1, wherein said measured data being preprocessed and preformatted prior to filtering said measured signal data.

5. A computer program for controlling a mechanical ventilator apparatus, wherein said program operate on signals obtained from af least two sensing means for measuring flow and pressure of breathing gas to a patient (1), said program using a delta energy term deduced from data indicative of flow changes of breathing obtained from said flow measurement in a pressure regulating procedure, and said program transmitting control signals for keeping a constant pressure to a mechanical ventilator in response to changes of the pressure of breathing gas using said flow change data as a pressure value compared against a set demand pressure value together with said measured pressure.

6. The computer program according to claim 5, wherein said computer program is arranged to filter said flow signals prior to analyzing said flow data for flow changes in order to reduce noise.

7. The computer program according to claim 5, further arranged to operate on flow signals obtained from said sensing means (10) being arranged at a mechanical ventilator side of tubing (3) for supplying breathing gas.

8. The computer program according to claim 6, wherein said measured data is preprocessed and preformatted prior to filtering said measured signal data.

## Patentansprüche

1. Eine mechanische Lüftervorrichtung (4) zum Zuführen von Atemgas an Menschen (1), wobei der Lüfter umfasst:
- eine Lüfteranordnung (4);
- eine Verarbeitungseinheit (201); und
- Eingabemittel zum Erhalten von Signalen (5, 6, 7, 8, 9, 10) die zumindest auf den Atemgasfluss und -druck schließen lassen;
wobei die Verarbeitungseinheit (201) angeordnet ist, die Flusssignale zu analysieren und Flussänderungswerte zu bestimmen, **dadurch gekennzeichnet, dass**
die Verarbeitungseinheit überdies angeordnet ist, aus den Flussänderungen einen Delta-Energieterm zu bestimmen, den Delta-Energieterm als Eingabe in einem druckregulierenden Verfahren zu verwenden und den Druck in Bezug auf einen Solldruckwert zu regulieren, wodurch ein konstanter Druckpegel geschaffen wird.

2. Die Vorrichtung nach Anspruch 1, wobei die Verarbeitungseinheit (201) angeordnet ist, vor dem Analysieren der Flusssignale die Flusssignale in einem rauschreduzierenden Filter, wie einem Tiefpassfilter, zu filtern.

3. Die Vorrichtung nach Anspruch 1, wobei das Erfassungsmittel zum Erfassen von Flusssignalen an einer Schlauchseite (3) des mechanischen Lüfters zum Zuführen von Atemgas angeordnet ist.

4. Die Vorrichtung nach Anspruch 1, wobei die gemessenen Daten vor dem Filtern der gemessenen Signaldaten vorverarbeitet und vorformatiert werden.

5. Ein Computerprogramm zum Steuern einer mechanischen Lüftervorrichtung, wobei das Programm aufgrund von Signalen arbeitet, die von mindestens zwei Erfassungsmitteln zum Messen des Flusses und des Drucks von Atemgas an einen Patienten (1) erhalten werden, wobei das Programm einen Delta-Energieterm verwendet, der von Daten abgeleitet wird, die auf Flussänderungen des Atems schließen lassen, die von der Flussmessung in einem druckregulierenden Verfahren erhalten werden, und das Programm Steuersignale zum Aufrechterhalten eines konstanten Drucks an einen mechanischen Lüfter als Reaktion auf Änderungen des Atemgasdrucks unter Verwendung der Flussänderungsdaten als Druckwert verglichen mit einem Solldruckwert, zusammen mit dem gemessenen Druck, übersendet.

6. Das Computerprogramm nach Anspruch 5, wobei das Computerprogramm angeordnet ist, die Flusssignale vor dem Analysieren der Flussdaten auf Flussänderungen zu filtern, um das Rauschen zu reduzieren.

7. Das Computerprogramm nach Anspruch 5, überdies angeordnet, aufgrund von Flusssignalen zu arbeiten, die von dem Erfassungsmittel (10) erhalten werden, das an einer Schlauchseite (3) des mechanischen Lüfters zum Zuführen von Atemgas angeordnet ist.

8. Das Computerprogramm nach Anspruch 6, wobei die gemessenen Daten vor dem Filtern der gemessenen Signaldaten vorverarbeitet und vorformatiert werden.

## Revendications

1. Appareil ventilateur mécanique (4) pour fournir un gaz respirable aux êtres humains (1), le ventilateur comprenant :
- un dispositif de ventilateur (4) ;
- une unité de traitement (201) ; et
- des moyens d'entrée pour obtenir des signaux (5, 6, 7, 8, 9, 10) indicatifs d'au moins un flux et d'une pression du gaz respirable ; dans lequel ladite unité de traitement (201) est disposée de façon à analyser lesdits signaux de flux déterminant des valeurs de changement de flux, **caractérisé en ce que** l'unité de traitement est en outre disposée de façon à déterminer un terme d'énergie delta d'après les changements de flux, à utiliser ledit terme d'énergie delta comme entrée dans une procédure de régulation de pression, et à réguler ladite pression contre une valeur de demande de pression établie en créant un niveau de pression constant.

2. Appareil selon la revendication 1, dans lequel ladite unité de traitement (201) est disposée de façon à filtrer lesdits signaux de flux dans un filtre réducteur de bruit, comme un filtre passe-bas, avant l'analyse desdits signaux de flux.

3. Appareil selon la revendication 1, dans lequel lesdits moyens de détection pour détecter les signaux de flux sont disposés sur un côté de tubage (3) du ventilateur mécanique pour fournir le gaz respirable.

4. Appareil selon la revendication 1, dans lequel lesdites données mesurées sont prétraitées et pré-formatées avant la filtration desdites données de signal mesurées.

5. Programme informatique pour commander un appareil ventilateur mécanique, dans lequel ledit programme fonctionne sur des signaux obtenus d'au moins deux systèmes de détection pour mesurer le flux et la pression du gaz respirable amené à un patient (1), ledit programme utilisant un terme d'énergie delta déduit des données indicatives des changements de flux de respiration obtenues d'après ladite mesure de flux dans une procédure de régulation de pression, et ledit programme transmettant des signaux de commande pour maintenir une pression constante à un ventilateur mécanique, en réponse aux changements de la pression du gaz respirable en utilisant lesdites données de changement de flux comme une valeur de pression par rapport à une valeur de pression de demande établie avec ladite pression mesurée.

6. Programme informatique selon la revendication 5, dans lequel ledit programme informatique est disposé de façon à filtrer lesdits signaux de flux avant l'analyse desdites données de flux pour les changements de flux, afin de réduire le bruit.

7. Programme informatique selon la revendication 5, disposé en outre de façon à fonctionner sur lesdits signaux de flux obtenus par le biais desdits moyens de détection (10), étant disposés sur un côté de tubage (3) du ventilateur mécanique pour alimenter le gaz respirable.

8. Programme informatique selon la revendication 6, dans lequel lesdites données mesurées sont prétraitées et pré-formatées avant de filtrer lesdites données de signal mesurées.
